# EUROPEAN PATENT APPLICATION

(11) **EP 3 954 331 A1**
(43) Date of publication of application: **16.02.2022**
(21) Application number: 20788292.9
(22) Date of filing: 03.04.2020
(51) Int. Cl.: A61F 2/24

(54) **CONNECTING STRUCTURE OF STENT AND VALVE LEAFLET AND INTERVENTIONAL VALVE-IN-VALVE AND INTERVENTIONAL AORTIC VALVE APPLYING CONNECTING STRUCTURE**

(30) Priority: 08.04.2019 CN 201910274761
(71) Applicant: Beijing Balance Medical Technology Co., Ltd., Beijing 102200 (CN)
(72) Inventor: JIN, Lei, Beijing 102200 (CN); MU, Hong, Beijing 102200 (CN); FAN, Zhihao, Beijing 102200 (CN)
(74) Representative: Murgitroyd & Company
(86) International application number: PCT/CN2020/083088
(87) International publication number: WO 2020/207332

(57) **Abstract**

The invention relates to a connecting structure of a stent and a valve leaflet, wherein the stent is a metal mesh tube, the valve leaflets are three fan-shaped valve leaflets arranged on the inner side of the stent, each of the three fan-shaped valve leaflets is provided with a free edge, an arc-shaped bottom edge and valve leaflet junction connecting parts extending on two sides, three connecting posts are uniformly distributed on the metal mesh tube, the junction connecting part of the fan-shaped valve leaflets comprises a radial overturning connecting part and an axial overturning connecting part, the radial overturning connecting part of each fan-shaped valve leaflet penetrates through the connecting post to penetrate from the inner side to the outer side then folds, after the inner side of the connecting post is folded, the axial overturning connecting part is connected and fixed to the connecting post through a suture. The invention also provides an interventional valve-in-valve or interventional aortic valve applying the connecting structure. The connecting structure of the stent and the valve leaflets can avoid stress concentration when the valve leaflets are opened and closed and friction generated between the stent and the valve leaflets, so that hemodynamic effects similar to bioprosthetic valves and similar durability functions are realized.

## Description

### Technical Field

The invention relates to the technical field of medical instruments, in particular to a connecting structure of a stent and a valve leaflet for interventional valve-in-valve or interventional aortic valve, and an interventional valve-in-valve and an interventional aortic valve applying the connecting structure.

### Background Art

Interventional valve-in-valve is a special re-interventional therapy for patients who can't undergo surgical valve replacement after valve destruction of previously implanted (interventional) artificial bioprosthetic heart valves for various reasons. That is, the interventional valve-in-valve is placed into the artificial bioprosthetic valve which has failed through the catheter to replace the failed bioprosthetic valve to achieve the purpose of treatment.

The left ventricular function of patients with severe aortic valve disease is seriously damaged, the quality of life of patients is reduced, the survival time is obviously shortened, and effective treatment is necessary. The incidence of aortic valvular disease due to aortic valve degeneration in the elderly over 65 reached 10%. With the aging of society, the proportion of aortic valvular disease is higher and higher, calculating by the fact that 85,000 of 35,000,000 65-year-old people in the United States need to change valves, there should be more than 200,000 old people waiting for treatment in China each year. In accordance with the guidelines issued by AHC/ACC in 2017, the age of patients with surgical implants of biological valves should be reduced to 50, and for patients of any age, bioprosthetic valve are recommended for contraindicated, inappropriate or undesirable anticoagulant therapy. In the future, the artificial bioprosthetic valve for aortic valve replacement in the elderly will increase year by year, and for those elderly and severe patients who cannot undergo valve replacement surgery, re-interventional therapy through the valve-in-the-valve will become the hope of final treatment. Having an interventional valve-in-valve with the same durability as a surgical bioprosthetic valve will be a sharp weapon for these patients to be treated again.

### Summary of the Invention

The technical problem to be solved by the present invention is to provide a connecting structure of a stent and valve leaflet for interventional valve-in-valve or interventional aortic valve, and an interventional valve-in-valve and an interventional aortic valve using the connecting structure, wherein a cushioning portion is arranged on the inner side of a metal stent, so that the valve leaflets can be prevented from being in direct contact with the stent, and the service durability of a valve product is improved.

The technical scheme adopted by the invention is to provide a connecting structure of a stent and valve leaflets for an interventional valve-in-valve or an interventional aortic valve, wherein the stent is a metal mesh tube, the valve leaflets are three fan-shaped valve leaflets arranged on the inner side of the stent, each of the three fan-shaped valve leaflets is provided with a free edge, an arc-shaped bottom edge and valve leaflet junction connecting parts extending on two sides, and three connecting posts are uniformly distributed on the metal mesh tube, each connecting post is at least provided with a rectangular slit, the junction connecting part of the fan-shaped valve leaflets comprises a radial overturning connecting part and an axial overturning connecting part, the radial overturning connecting part of each fan-shaped valve leaflet penetrates through the rectangular slit of the connecting post to penetrate from the inner side to the outer side then folds, the axial overturning connecting part is folded on the inner side of the connecting post to forms a cushioning portion, and then is connected and fixed to the connecting post through a suture. Further, the radial overturning connecting parts of the two adjacent valve leaflets junction connecting parts are connected through a flexible connecting piece in a suturing mode, then penetrate through the rectangular slit of the connecting post, and are fixed with the connecting post through a suture after clamping and fixing with a rigid gasket on the inner side of the flexible connecting piece.

Further, holes or rectangular frames are symmetrically arranged on two sides of the rectangular slit on each connecting post, the number of the holes is four to eight, and the number of the rectangular frames is two or four.

Furthermore, the material of the stent is an implantable alloy material, the implantable alloy material is a cobalt-based alloy, a nickel-titanium alloy or a stainless steel material, and the material of the valve leaflet is an animal-derived tissue material or a medical polymer material. The invention also provides an interventional valve-in-valve or an interventional aortic valve applying the above connecting structure, which comprises a sent that can be radially compressible and in a slightly flaring shape after being expanded by a balloon, three fan-shaped valve leaflets arranged on the inner side of the stent, the three fan-shaped valve leaflets are respectively provided with a free edge, an arc-shaped bottom edge and valve leaflet junction connecting parts extending on the two sides, the stent is a metal mesh tube, and three connecting posts are uniformly distributed on the metal mesh tube; each connecting post is at least provided with a rectangular slit, the junction connecting part of the fan-shaped valve leaflets comprises a radial overturning connecting part and an axial overturning connecting part, and the radial overturning connecting part of each fan-shaped valve leaflet penetrates through the rectangular slit of the connecting post and penetrates from the inner side to the outer side then folds; and the axial overturning connecting part is folded at the inner side of the connecting post to form a cushioning portion, then connected and fixed to the connecting post through a suture, and a coating membrane is arranged on the body wall of the stent.

Further, the radial overturning connecting parts of the two adjacent valve leaflets junction connecting parts are connected through a flexible connecting piece in a suturing mode, then penetrate through the rectangular slit of the connecting post, and are fixed with the connecting post through a suture after clamping and fixing with a rigid gasket on the inner side of the flexible connecting piece.

Further, holes or rectangular frames are symmetrically arranged on two sides of the rectangular slit on each connecting post, the number of the holes is four to eight, and the number of the rectangular frames is two or four. Furthermore, the material of the stent is an implantable alloy material, the implantable alloy material is a cobalt-based alloy, a nickel-titanium alloy or a stainless steel material, and the material of the valve leaflet is an animal-derived tissue material or a medical polymer material.

Furthermore, the stent is provided with a plurality of columns of axial supporting rods arranged between the connecting posts, three rows of transversely extending circumferential supporting rods are arranged between the connecting posts and the axial supporting rods, the lower first row of circumferential supporting rods define the inflow end of the stent, the second row of circumferential supporting rods and the third row of circumferential supporting rods spaced from the first row define an outflow end of the stent, and each row of circumferential supporting rods consists of a plurality of groups of angular supporting rods connected together; each group of supporting rods is in the shape of a deformable V, the deformation angle is 0-90 degrees, each group of circumferential supporting rods in the first row and the second row are arranged in parallel and opposite to the direction of each group of circumferential supporting rods in the third row, and a coating membrane on the body wall of the stent is sewn between the first row of circumferential supporting rods and the second row of circumferential supporting rods. Furthermore, the stent is provided with four rows of transversely extending circumferential supporting rods and a plurality of columns of axial supporting rods arranged between the circumferential supporting rods, wherein the lower first and second row of circumferential supporting rods define the inflow end of the stent, the third and fourth row of circumferential supporting rods define the outflow end of the stent, each row of circumferential supporting rods consists of a plurality of groups of angular supporting rods connected together, and each group of supporting rods is in a deformable V shape; the deformation angle is 0-90 degrees, a plurality of columns of axial supporting rods and a plurality of groups of circumferential supporting rods are mutually connected to form a honeycomb space, and a coating membrane on the body wall of the stent is sewn between the first row of circumferential supporting rods and the third row of circumferential supporting rods.

Further, a coating membrane is sewn between the first row of circumferential supporting rods and the second row of circumferential supporting rods outside the body wall of the stent.

Further, the angle between the outer edge of the balloon-expanded stent and its axis is between 0° and 30°.

The invention has the beneficial effects that: the invention provides a connecting structure of a stent and valve leaflets for interventional valve-in-valve or interventional aortic valve, and an interventional valve-in-valve and an interventional aortic valve applying the connecting structure. Due to the fact that the animal-derived tissue material is folded at the inner side of the valve leaflet junction connection tissue to form a cushioning portion, and then the cushioning portion is connected and fixed on the connecting post of the stent through sutures, the valve leaflets can be prevented from being rubbed or scratched by the metal stent in the opening and closing processes; and due to the fact that the connecting posts in the connecting structure are double rows of holes or rectangular frame routing, the sutures at the seams of the junction connecting parts of the valve leaflets are fully fixed with the holes or the rectangular frame, stress concentration of the valve leaflets in the opening and closing processes can be avoided, use durability of the interventional valve-in-valve or the interventional aortic valve is improved, and a durable effect equivalent to that of a surgical valve is achieved. The interventional valve-in-valve in the present invention refers to the accumulation of research and clinical application of the artificial bioprosthetic heart valve for more than 50 years, and under the same condition of the bioprosthetic valve chemical modification technology, the structural design of the interventional valve, the connection and fixation between the valve leaflet boundary tissues and he connection and fixation of the valve leaflet tissues and the set structure of the stent are realized, so that the requirements of hydromechanics and valve firmness of valve during opening and closing are met.

### Brief Description of the Drawings

FIG. 1 is a schematic view of an artificial heart surgical valve structure;
FIG. 2 is a schematic view of an interventional valve-in-valve structure for re-interventional therapy with an artificial heart valve according to an example of the present invention;
FIG. 3 is a valve leaflet deployment plan view of an interventional valve-in-valve for re-interventional therapy of an artificial bioprosthetic heart valve according to an example of the present invention;
FIGS. 4A-C are side and perspective views of the folding suture of three valve leaflets at junction connecting part commissures of an interventional valve-in-valve for re-interventional therapy of an artificial bioprosthetic heart valve according to an example of the present invention;
FIG. 5 is an expanded plan view showing a polyester rim coated for reinforcement at the curved bottom edge of a valve leaflet of an interventional valve-in-valve for re-interventional therapy of an artificial bioprosthetic heart valve according to an example of the present invention;
FIG. 6 is a perspective view of a metal stent of an interventional valve-in-valve for re-interventional therapy with an artificial heart valve according to an example of the present invention;
FIG. 7 is a perspective view of a valve in an interventional valve-in-valve for re-interventional therapy of an artificial bioprosthetic heart valve according to an example of the present invention after being dilated by a balloon;
FIG. 8 is a top plan view of an interventional valve for re-interventional therapy of an artificial bioprosthetic heart valve according to an example of the present invention when the valve is closed;
FIG. 9 is a top plan view of an interventional valve for re-interventional therapy of an artificial bioprosthetic heart valve according to an example of the present invention when the valve is open;
FIG. 10 is a perspective view of a metal stent of an interventional aortic valve for interventional therapy with an artificial heart valve according to another example of the present invention;
FIG. 11 is a schematic view of an interventional aortic valve structure for re-interventional therapy with an artificial heart valve according to another example of the present invention;
FIGS. 12A-B are schematic views illustrating the manner in which connecting posts and valve leaflets are connected according to an example of the present invention.

### Detailed Description of the Invention

The present invention provides a connecting structure of a stent and a valve leaflet for an interventional valve-in-valve or an interventional aortic valve, and an interventional valve-in-valve and an interventional aortic valve using the connecting structure. The following detailed description illustrates specific embodiments with the understanding that the specific embodiments described herein are merely illustrative of the invention and are not intended to be limiting thereof.

### Example 1: interventional valve-in-valve

The interventional valve-in-valve has a "lower" end and an "upper" end. In the context of this application, the terms "lower" and "upper" are used interchangeably with the terms "inflow" and "outflow", respectively. Thus, for example, the lower end of the interventional valve-in-valve is its inflow end and the upper end of the interventional valve-in-valve is its outflow end.

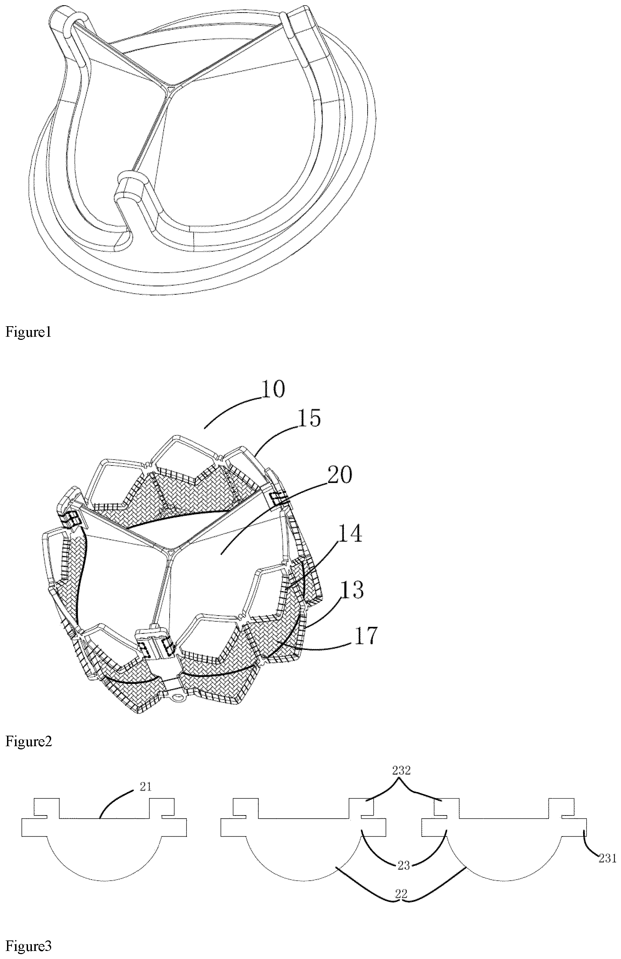

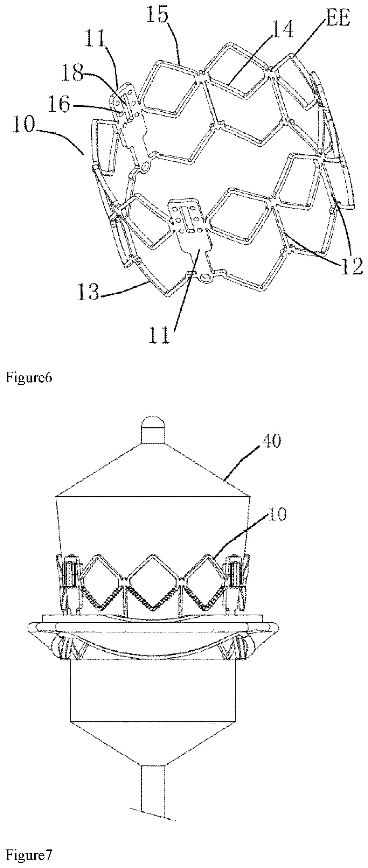

Referring to FIG. 2, there is shown a schematic view of an interventional valve-in-valve structure for re-interventional therapy of an artificial bioprosthetic heart valve according to an example of the present invention, comprising a stent 10 that can be radially compressible and in a slightly flaring shape after being expanded by a balloon (referring to FIG. 7), three fan-shaped valve leaflets 20 arranged on the inner side of the stent 10, the three fan-shaped valve leaflets 20 are respectively provided with a free edge 21, an arc-shaped bottom edge 22 and valve leaflet junction connecting parts 23 extending on the two sides (referring to FIG. 3). Referring further to FIG. 6, the stent is a metal mesh tube, wherein three connecting posts 11 are uniformly distributed on the metal mesh tube, six columns of axial supporting rods 12 are uniformly distributed among the three connecting posts 11, three rows of circumferential supporting rods 13, 14 and 15 transversely penetrating and extending are arranged between the connecting posts 11 and the six columns of axial supporting rods 12, and the lower first row of circumferential supporting rods 13 defines the inflow end of the stent 10, the second row of circumferential supporting rods 14 and the third row of circumferential supporting rods 15 spaced from the first row of circumferential supporting rods 13 define the outflow end of the stent. Each row of circumferential supporting rods is formed by connecting supporting rods EE with multiple constituent angles, each group of supporting rods EE is in the shape of a deformable V, the deformation angle is 0-90 degrees, and each group of circumferential supporting rods of the first row of circumferential supporting rods 13 and the second row of circumferential supporting rods 14 are arranged in parallel and opposite to the direction of each group of circumferential supporting rods of the third row of circumferential supporting rods 15 to form a plurality of grids which can be synchronously deformed. The stent made of the metal mesh tube can adapt to a use mode that an interventional valve-in-valve is longitudinally compressed and then expanded during interventional therapy. Further, the connecting post 11 is provided with a rectangular slit 18, two rows of six holes 16 are symmetrically formed in two sides of the rectangular slit 18 for fixedly connecting the fan-shaped valve leaflets through sutures. In the plurality of grids of the stent 11, a coating membrane 17 is sewn between the first row of circumferential supporting rods 13 and the second row of circumferential supporting rods 14. The coating membrane on the body wall of the stent is sewn between the first row of circumferential supporting rods and the third row of circumferential supporting rods. In some cases, an outside coating membrane (not shown) is further sewn to the outside at the same location as desired. The material of the coating membrane can be animal-derived tissues or medical polymer materials which would occur to a person skilled in the art.

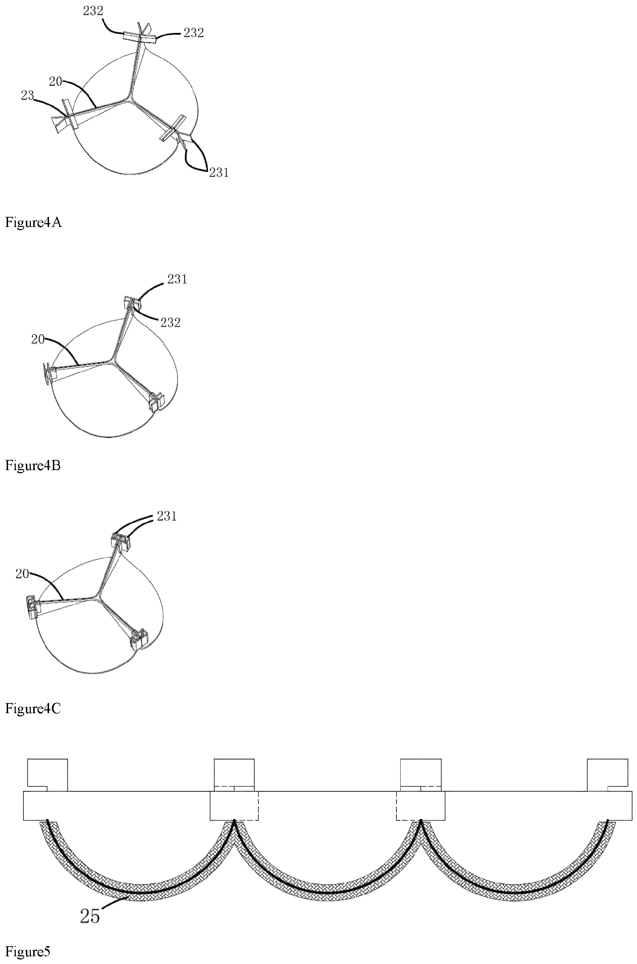

Referring again to FIGS. 2 and 4A-C, FIG. 5, the junction connecting part 23 of the fan-shaped valve leaflet 20 includes two portions: a radial overturning connecting part 231 and an axial overturning connecting part 232, wherein the radial overturning connecting part 231 is aligned in pairs at first, and then penetrates the rectangular slit 18 from the inner side of the connecting column 11 to be opened and folded, and fixes to the connecting column through sutures, the axial overturning connecting part 232 is then folded down on the inside of the connecting post in the opposite direction to fit and align with the fan-shaped valve leaflets 20 by using sutures, such that a section of cushioning portion 24 is formed at the root of the free edge of the fan-shaped valve leaflet, and the cushioning portion 24 is further secured to the hole 16 of connecting post 11 of the stent by sutures. The radial overturning connecting part may be re-fixed to the extent necessary to overturn to wrap around the connecting post 11. The arc-shaped bottom edge of the fan-shaped valve leaflet can also be coated with a reinforced coating membrane 25, and the coating membrane further sewn on the body wall of the stent through the coating membrane 25. Thus, an interventional valve-in-valve according to examples of the present invention is formed.

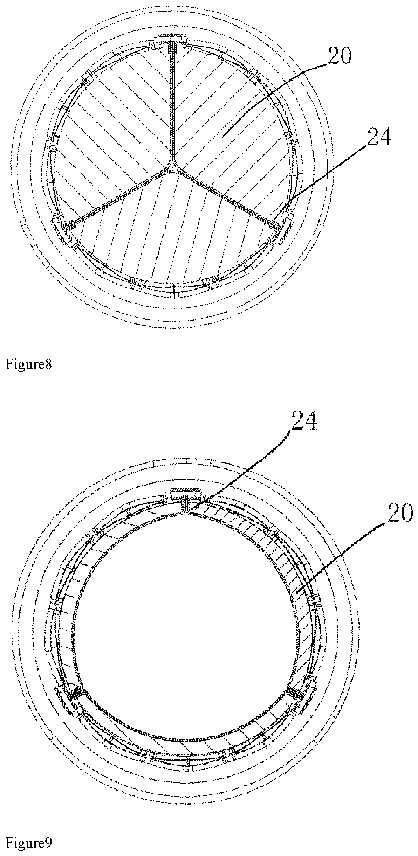

Referring to FIGS. 8 and 9, when the valve leaflets of the interventional valve-in-valve are opened, due to the existence of the cushioning portion 24, the valve leaflets can be prevented from being rubbed or scratched by the metal stent in the opening and closing processes; and due to the fact that the connecting posts in the connecting structure are double rows of holes routing, the sutures at the seams of the junction connecting parts of the valve leaflets are fully fixed with the holes or the rectangular frame, stress concentration of the valve leaflets in the opening and closing processes can be avoided, use durability of the interventional valve-in-valve or the interventional aortic valve is improved, and a durable effect equivalent to that of a surgical valve is achieved. The interventional valve-in-valve of the present invention can be used for the re-interventional therapy of the interventional valve in addition to the re-interventional therapy of the surgical valve in this example.

### Example 2: interventional aortic valve

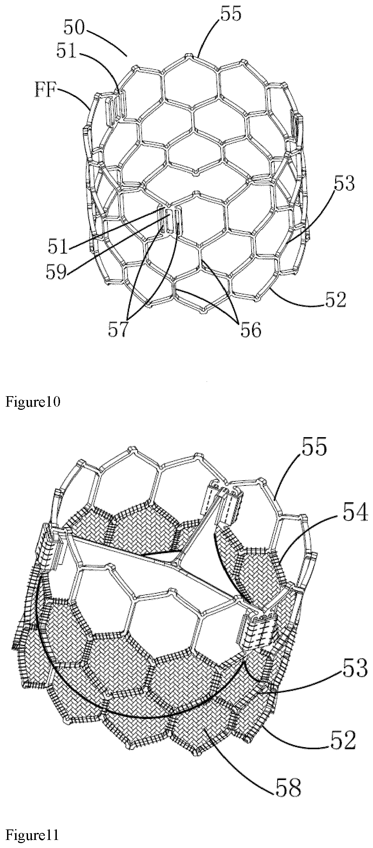

The structure of the present invention can be directly used for the interventional therapy of the aortic valve in addition to the re-interventional therapy in Example 1. The interventional aortic valve of the example has a stent structure different from that of Example 1, and other structures are substantially the same as those of Example 1. In some cases, a larger outflow end and a larger stent height are required. Referring to FIGS. 10 and 11, in this example, the stent 50 is a metal mesh tube having three connecting posts 51 evenly distributed thereon, the stent is provided with four rows of transversely extending circumferential supporting rods 52, 53, 54 and 55, and a plurality of axial supporting rods 56 arranged between the circumferential supporting rods, wherein the lower first and second row of circumferential supporting rods 52, 53 define the inflow end of the stent, the third and fourth row of circumferential supporting rods 54, 55 define the outflow end of the stent, each row of circumferential supporting rods consists of a plurality of groups of angular supporting rods EE connected together, and each group of supporting rods is in a deformable V shape; the deformation angle is 0-90 degrees, the plurality of columns of axial supporting rods 56 and a plurality of groups of circumferential supporting rods are mutually connected to form a deformable honeycomb grid. Wherein, the cellular grid in which the outflow end is defined is larger. Furthermore, the upper portion of the connecting post 11 is provided with a rectangular slit 59, two rows of rectangular frames 57 are symmetrically arranged on two sides of the slit, in the example, after the axial overturning connecting parts of the adjacent fan-shaped valve leaflets are aligned, the adjacent fan-shaped valve leaflets firstly pass through the rectangular slit 59, then are opened to pass through the two rectangular frames 57 respectively, followed by being overturned again to cover the outer sides of the connecting post, and are fixed to the connecting post through sutures, so as to improve the connection strength of valve leaflet and stent. In the plurality of grids of the stent 51, a coating membrane 58 is sewn between the first row of circumferential supporting rods 52 and the third row of circumferential supporting rods 54. In some cases, an outer coating membrane (not shown) is also sewn to the outside of the coating membrane between the first row of circumferential supporting rods 52 and the second row of circumferential supporting rods 53, as desired. The material of the coating membrane can be animal-derived tissues or medical polymer materials which would occur to a person skilled in the art.

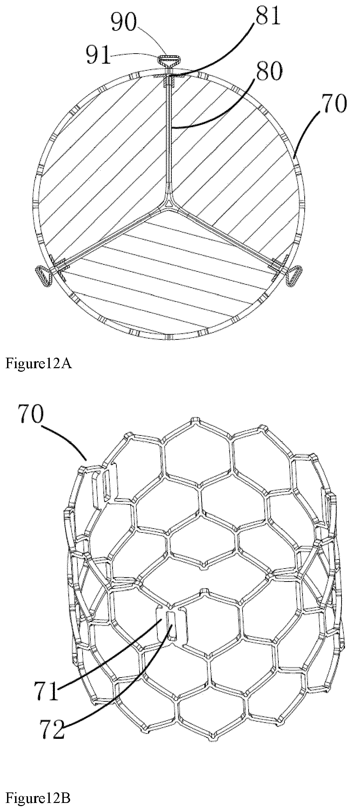

As shown in FIGS. 12A-B, in some cases, it is also possible to use such a manner that the valve leaflets 80 are fixedly connected to the stent 70, the connecting post 71 of the stent 70 has only one slit 72, a flexible connecting piece 90 is sewn between the axial overturning connecting part 81 of the two adjacent valve leaflets junction, penetrates through the rectangular slit 72, then the reinforcing gasket 91 is placed, and is fully fixedly connected to the connecting post through sutures to enclosed into a whole. The radial overturning connecting part of the valve leaflets may take the same way as in the previous examples.

The stent in all examples may be implemented as, but is not limited to, a cobalt-based alloy or nickel-titanium alloy or stainless steel material or other implantable alloy material stent or the like, and is not specifically limited thereto; the valve leaflet is an animal-derived tissue material or a medical polymer material, for example, any one of a porcine pericardium, a bovine pericardium or a sheep pericardium tissue material or any one of medical polymer materials, and is not specifically limited thereto. The suture is any one of medical polymer materials.

Finally, it should be noted that: the above examples are merely illustrative of the technical solutions of the present invention and are not intended to be limiting thereof; although the present invention has been described in detail with reference to the foregoing embodiments, those skilled in the art will appreciate that: the technical solutions of the above-mentioned embodiments can still be modified, or some or all of the technical features thereof can be equivalently replaced; these modifications or replacements do not cause the essence of the corresponding technical solutions to deviate from the scope of the technical solutions of the embodiments of the present invention.

## Claims

1. A connecting structure of a stent and a valve leaflet for an interventional valve-in-valve or an interventional aortic valve, wherein the stent is a metal mesh tube, the valve leaflets are three fan-shaped valve leaflets arranged on the inner side of the stent, each of the three fan-shaped valve leaflets is provided with a free edge, an arc-shaped bottom edge and valve leaflet junction connecting parts extending on two sides, **characterized in that** three connecting posts are uniformly distributed on the metal mesh tube, each connecting post is at least provided with a rectangular slit, the junction connecting parts of the fan-shaped valve leaflets comprise a radial overturning connecting part and an axial overturning connecting part, the radial overturning connecting part of the each fan-shaped valve leaflet penetrates through the rectangular slit of the connecting post to penetrate from the inner side to the outer side then folds, the axial overturning connecting part is folded on the inner side of the connecting post to forms a cushioning portion, and then is connected and fixed to the connecting post through a suture.

2. The connecting structure of the stent and the valve leaflet for the interventional valve-in-valve or the interventional aortic valve of claim 1, **characterized in that** the radial overturning connecting parts of the two adjacent valve leaflets junction connecting parts are further connected through a flexible connecting piece in a suturing mode, then penetrate through the rectangular slit of the connecting post, and are fixed with the connecting post through a suture after clamping and fixing with a rigid gasket on the inner side of the flexible connecting piece.

3. The connecting structure of the stent and the valve leaflet for the interventional valve-in-valve or the interventional aortic valve of claim 1, **characterized in that** holes or rectangular frames are symmetrically arranged on two sides of the rectangular slit on each connecting post, the number of the holes is four to eight, and the number of the rectangular frames is two or four.

4. The connecting structure of the stent and the valve leaflet for the interventional valve-in-valve or interventional aortic valve of claim 1, **characterized in that** the material of the stent is an implantable alloy material, the implantable alloy material is a cobalt-based alloy, a nickel-titanium alloy or a stainless steel material, and the material of the valve leaflet is an animal-derived tissue material or a medical polymer material.

5. An interventional valve-in-valve or interventional aortic valve employing the connecting structure of the stent and the valve leaflet of any one of claims 1-4, comprising a sent that can be radially compressible and in a slightly flaring shape after being expanded by a balloon, three fan-shaped valve leaflets arranged on the inner side of the stent, the three fan-shaped valve leaflets are respectively provided with a free edge, an arc-shaped bottom edge and valve leaflet junction connecting parts extending on the two sides, **characterized in that** the stent is a metal mesh tube, and three connecting posts are uniformly distributed on the metal mesh tube, each connecting post is at least provided with a rectangular slit, the junction connecting parts of the fan-shaped valve leaflets comprise a radial overturning connecting part and an axial overturning connecting part, the radial overturning connecting part of each fan-shaped valve leaflet penetrates through the rectangular slit of the connecting post to penetrate from the inner side to the outer side then folds, the axial overturning connecting part is folded on the inner side of the connecting post to forms a cushioning portion, and then is connected and fixed to the connecting post through a suture, the body wall of the stent is provided with a coating membrane.

6. The interventional valve-in-valve or the interventional aortic valve of claim 5, **characterized in that** the stent is provided with a plurality of columns of axial supporting rods arranged between the connecting posts, three rows of transversely extending circumferential supporting rods are arranged between the connecting posts and the axial supporting rods, the lower first row of circumferential supporting rods define an inflow end of the stent, the second row of circumferential supporting rods and the third row of circumferential supporting rods spaced from the first row of circumferential supporting rods define an outflow end of the stent, and each row of circumferential supporting rods consists of a plurality of groups of angular supporting rods connected together; each group of supporting rods is in the shape of a deformable V, the deformation angle is 0-90 degrees, each group of circumferential supporting rods in the first row and the second row are arranged in parallel and opposite to the direction of each group of circumferential supporting rods in the third row, and the coating membrane on the body wall of the stent is sewn between the first row of circumferential supporting rods and the second row of circumferential supporting rods.

7. The interventional valve-in-valve or the interventional aortic valve of claim 5, **characterized in that** the stent is provided with four rows of transversely extending circumferential supporting rods and a plurality of columns of axial supporting rods arranged between the circumferential supporting rods, wherein the lower first and second row of circumferential supporting rods define the inflow end of the stent, the third and fourth row of circumferential supporting rods define the outflow end of the stent, each row of circumferential supporting rods consists of a plurality of groups of angular supporting rods connected together, and each group of supporting rods is in a deformable V shape; the deformation angle is 0-90 degrees, the plurality of columns of axial supporting rods and the plurality of groups of circumferential supporting rods are mutually connected to form a honeycomb space, and the coating membrane on the body wall of the stent is sewn between the first row of circumferential supporting rods and the third row of circumferential supporting rods.

8. The interventional valve-in-valve or the interventional aortic valve of claim 5, **characterized in that** a coating membrane is sewn between the first row of circumferential supporting rods and the second row of circumferential supporting rods outside the body wall of the stent.

9. The interventional valve-in-valve or the interventional aortic valve of claim 5, **characterized in that** the angle between the outer edge of the balloon-expanded stent and the axis thereof is between 0°and 30°.
